# EUROPÄISCHE PATENTSCHRIFT

(11) **EP 3 381 488 B1**
(45) Veröffentlichungstag und Bekanntmachung des Hinweises auf die Patenterteilung: **27.01.2021**
(21) Anmeldenummer: 18161565.9
(22) Anmeldetag: 13.03.2018
(51) Int. Cl.: A61M 5/14

(54) **SICHERUNGSVORRICHTUNG ZUR SICHERUNG EINES INFUSIONSGERÄTS**
DEVICE FOR SECURING AN INFUSION APPARATUS
DISPOSITIF DESTINÉ À SÉCURISER UN APPAREIL DE PERFUSION

(30) Priorität: 28.03.2017 DE 102017205188
(43) Veröffentlichungstag der Anmeldung: 03.10.2018
(73) Patentinhaber: B. Braun Melsungen AG, 34212 Melsungen (DE)
(72) Erfinder: Fuchs, Jürgen, 34308 Bad Emstal (DE)
(74) Vertreter: Patentanwälte Ruff, Wilhelm, Beier, Dauster & Partner mbB

(56) Entgegenhaltungen:
- WO-A1-2011/118411
- WO-A1-2014/000494
- WO-A1-2014/008832
- CN-A- 104 758 995
- CN-U- 201 719 627
- CN-Y- 201 426 880
- DE-U1- 20 302 788
- JP-A- H07 124 264
- JP-A- H09 239 026
- US-A- 4 589 171

## Beschreibung

Die Erfindung betrifft eine Sicherungsvorrichtung nach dem Oberbegriff von Anspruch 1.

Infusionsgeräte und Infusionsbehälter sind im Bereich der Medizintechnik allgemein bekannt. Solche Infusionsgeräte werden auch als Infusionssysteme bezeichnet und dienen zur flüssigkeitsleitenden Verbindung eines Infusionsbehälters mit einem patientenseitigen Zugang. Durch eine derartige Leitungsverbindung kann Infusionsflüssigkeit aus dem Infusionsbehälter in den patientenseitigen Zugang eingeleitet werden, beispielweise im Rahmen einer Druck- oder Schwerkraftinfusionstherapie. Bekannte Infusionsgeräte weisen zur Fixierung an dem Infusionsbehälter einen Einstechdorn zum Einstechen in einen Einstechbereich des Infusionsbehälters und zur Ausleitung der Infusionsflüssigkeit aus diesem sowie eine an den Einstechdorn stirnseitig angrenzende Tropfkammer auf. Letztere dient zur tropfenweisen Aufnahme der mittels des Einstechdorns aus dem Infusionsbehälter ausgeleiteten Infusionsflüssigkeit. Zudem kann das Infusionsgerät eine zwischen dem Einstechdorn und der Tropfkammer angeordnete Belüftungseinrichtung zur kontrollierten Belüftung des Infusionsbehälters, eine der Tropfkammer nachgeordnete Fluidleitung zur Ableitung der Infusionsflüssigkeit aus der Tropfkammer, eine mit der Fluidleitung in einer Wirkverbindung stehende Stelleinrichtung zur Einstellung des Flüssigkeitsdurchflusses durch die Fluidleitung sowie einen ausgangsseitig an der Fluidleitung angeordneten Anschlusskonnektor zur Verbindung der Fluidleitung mit dem patientenseitigen Zugang umfassen. Bekannte Infusionsbehälter weisen eine Flaschen- oder Beutelform auf und sind zur Aufnahme einer Infusionsflüssigkeit oder -lösung vorbereitet. Solche Infusionsbehälter weisen einen Einstechbereich auf, der mittels eines Einstechdorns eines Infusionsgeräts durchstochen werden kann. Bei allgemein bekannten Infusionsbehältern kann der Einstechbereich an einem elastischen Stopfen vorgesehen sein, der zudem als Verschluss des Infusionsbehälters dienen kann. Zur Ausleitung von Infusionsflüssigkeit aus dem Infusionsbehälter wird mittels des Einstechdorns des Infusionsgeräts der Einstechbereich von außen bis in das Innere des Infusionsbehälter hineinreichend durchstochen. Die Infusionsflüssigkeit kann derart durch eine längserstreckte Durchgangsbohrung des Einstechdorns in die Tropfkammer, weiter in die Fluidleitung und letztlich über den Anschlusskonnektor in den patientenseitigen Zugang geleitet werden. Hierbei werden das Infusionsgerät und der Infusionsbehälter durch den zwischen dem Einstechdorn und dem Einstechbereich wirkenden Reibschluss mechanisch miteinander verbunden und bilden derart eine Infusionsanordnung.

Aus der CN 104 758 995 A ist eine Sicherungsvorrichtung für eine solche Infusionsanordnung bekannt. Die bekannte Sicherungsvorrichtung weist ein erstes Befestigungselement und ein zweites Befestigungselement auf. Das erste Befestigungselement ist zur Befestigung an einem Halsbereich des Infusionsbehälters vorgesehen. Das zweite Befestigungselement ist zu dem ersten Befestigungselement beabstandet angeordnet und mit dem Infusionsgerät verbindbar. In verbundenem Zustand greift das zweite Befestigungselement an einem Abschnitt eines Einstechdorns des Infusionsgeräts an.

Aus der DE 203 02 788 U1 ist eine weitere Sicherungsvorrichtung bekannt, die in Form einer Klammer gestaltet und zur Sicherung eines Einstechdorns an einer Infusionsflasche vorgesehen ist. Die Klammer weist ein erstes gabelförmiges Befestigungselement und ein zweites gabelförmiges Befestigungselement auf. Das zweite Befestigungselement ist zur Verbindung mit einem Abschnitt des Einstechdorns vorgesehen.

Eine weitere Sicherungsvorrichtung ist aus der CN 201 426 880 Y bekannt. Die Sicherungsvorrichtung weist eine klammerartige Gestaltung auf. In einem Benutzungszustand der Sicherungsvorrichtung ist vorgesehen, dass diese einends mit einem Infusionsschlauch und andernends mit einem Halsbereich einer Infusionsflasche verbunden ist.

Aufgabe der Erfindung ist es, eine verbesserte Handhabbarkeit einer solchen Infusionsanordnung zu erreichen.

Diese Aufgabe wird durch eine Sicherungsvorrichtung mit den Merkmalen von Anspruch 1 gelöst. Die Erfindung geht von der Überlegung aus, dass eine auf übliche Weise mechanisch verbundene Infusionsanordnung nachteilig hinsichtlich ihrer Handhabbarkeit ist. Insbesondere kann einem ungewollten Trennen der Infusionsanordnung durch die im Wesentlichen reibschlüssige Verbindung zwischen Einstechdorn und Einstechbereich unter gewissen Umständen nicht ausreichend entgegengewirkt werden. Kommt es während einer Infusionstherapie zu einem solchen ungewollten Trennen, kann eine Beeinträchtigung des Patienten nicht ausgeschlossen werden. Die erfindungsgemäße Sicherungsvorrichtung bewirkt hingegen eine Sicherung der fixierten Anordnung in zumindest einer Sicherungsrichtung. Zu diesem Zweck sieht die Sicherungsvorrichtung ein erstes und ein zweites Befestigungselement vor. Diese beiden Befestigungselemente sind mittels eines Verbindungselements miteinander verbunden. Das erste Befestigungselement ist dem Befestigungsbereich des Infusionsbehälters zugeordnet und kann mit diesem mechanisch verbunden werden. Das zweite Befestigungselement ist dem Befestigungsbereich des Infusionsgeräts zugeordnet und kann mit diesem mechanisch verbunden werden. Zur Sicherung einer aus dem Infusionsbehälter und dem an diesem fixierten Infusionsgerät gebildeten Infusionsanordnung wird zumindest eines der Befestigungselemente mit dem ihm jeweils zugeordneten Befestigungsbereich verbunden. Unter einer Sicherung im Sinne der Erfindung ist ein Sichern einer bestehenden mechanischen Verbindung zu verstehen. Ein solches Sichern kann durch eine zusätzlich zu der bestehenden Verbindung gleichzeitig wirkende mechanische Verbindung oder durch eine passiv redundante, die bestehende Verbindung erst im Falle ihrer Trennung ersetzende mechanische Verbindung erreicht werden. Die Sicherungsvorrichtung bewirkt derart eine Trennsicherung in zumindest einer Sicherungsrichtung. Unter einer Sicherungsrichtung im Sinne der Erfindung ist eine Richtung zu verstehen, in welcher einem Trennen einer bestehenden mechanischen Verbindung zwischen dem Infusionsbehälter und dem Infusionsgerät entgegengewirkt wird. Soweit diese mechanische Verbindung im Wesentlichen durch einen Reibschluss zwischen dem Einstechdorn des Infusionsgeräts und dem Einstechbereich des Infusionsbehälters gebildet ist, verläuft die Sicherungsrichtung im Wesentlichen parallel zur Einstechrichtung des Einstechdorns in den Einstechbereich. Derart wirkt die Sicherungsvorrichtung erfindungsgemäß einem ungewollten Trennen der Verbindung zwischen dem Infusionsgerät und dem Infusionsbehälter, beispielsweise infolge eines unbeabsichtigten Herausziehens des Einstechdorns aus dem Einstechbereich, entgegen. Auf diese Weise kann eine Beeinträchtigung eines Patienten während der Infusionstherapie vermieden werden. Demzufolge wird letztlich eine deutlich verbesserte Handhabbarkeit der Infusionsanordnung erreicht.

Die erfindungsgemäße Lösung eignet sich, wie vorstehend beschrieben, in besonders vorteilhafter Weise zur Sicherung eines Infusionsgeräts an einem Infusionsbehälter. Die erfindungsgemäße Lösung kann aber auch zur Sicherung eines Transfusionsgeräts an einem Transfusionsbehälter und insoweit bei einer Transfusionstherapie eingesetzt werden.

In Ausgestaltung der Erfindung ist zumindest eines der Befestigungselemente derart gestaltet, dass es den jeweiligen Befestigungsbereich in einem befestigten Zustand zumindest abschnittsweise und im Wesentlichen quer zu der Sicherungsrichtung von außen umgreift. Derart kann eine Verbindung mit dem jeweiligen Befestigungsbereich manuell leicht zugänglich und insoweit besonders ergonomisch hergestellt werden. Zudem ist die derartige Verbindung gut einsehbar und demzufolge leicht überprüfbar. Vorteilhafterweise sind beide Befestigungselemente derart gestaltet, dass sie den jeweiligen Befestigungsbereich in einem befestigten Zustand zumindest abschnittsweise und im Wesentlichen quer zu der Sicherungsrichtung von außen umgreifen.

In weiterer Ausgestaltung der Erfindung ist zumindest eines der Befestigungselemente derart gestaltet, dass es formschlüssig mit dem jeweiligen Befestigungsbereich verbindbar ist. Formschlüssige Verbindungen können hochbeanspruchbar dimensioniert werden. Dies ist demnach eine besonders bauraum- und materialsparende Ausgestaltung der Erfindung. Vorteilhafterweise sind beide Befestigungselemente derart gestaltet, dass sie formschlüssig mit dem jeweiligen Befestigungsbereich verbindbar sind.

In weiterer Ausgestaltung der Erfindung weist zumindest eines der Befestigungselemente ein Rastelement auf, welches mit dem jeweiligen Befestigungsbereich kraft- und/oder formschlüssig verbindbar ist. In besonders vorteilhafter Weise ist das Rastelement zur Verbindung mit dem jeweiligen Befestigungsbereich elastisch aufweitbar, insbesondere quer zur Sicherungsrichtung. Zum Herstellen der Verbindung wird das Rastelement elastisch geweitet und an dem jeweiligen Befestigungsbereich festgelegt. Die der elastischen Aufweitung entgegenwirkende Federwirkung bewirkt ein Verrasten an dem Befestigungsbereich. Demzufolge kann eine besonders einfach herstellbare und zuverlässige Verbindung erreicht werden.

In weiterer Ausgestaltung der Erfindung ist das Rastelement ringförmig ausgebildet und weist zumindest einen längs seiner Umfangsrichtung offenen Wandbereich auf. Vorteilhafterweise ist das Rastelement C-förmig mit einem offenen Wandbereich ausgebildet.

Weiter gemäß der Erfindung weist zumindest eines der Befestigungselemente eine Profilierung auf, welche zur Aufnahme zumindest eines Abschnittes einer Profilierung des jeweiligen Befestigungsbereichs vorgesehen ist. Die Profilierungen können komplementär zueinander ausgebildet sein. Unter einer Profilierung im Sinne der Erfindung sind sowohl Vertiefungen eines Wandabschnittes als auch Aufragungen eines Wandabschnittes zu verstehen. In besonders vorteilhafter Weise weist zumindest eines der Befestigungselemente eine Nut auf, die zur Aufnahme zumindest eines Abschnittes einer im Wesentlichen senkrecht von dem Befestigungsbereich erstreckten Wandaufragung vorgesehen ist.

In weiterer Ausgestaltung der Erfindung ist das erste Befestigungselement derart gestaltet, dass es mit einem Halsbereich eines in Form einer Flasche ausgebildeten Infusionsbehälters verbindbar ist. Zu diesem Zweck ist das erste Befestigungselement vorteilhafterweise im Wesentlichen ringförmig ausgestaltet und weist einen offenen Wandabschnitt in radialer Richtung auf, wobei der Ringdurchmesser im Wesentlichen dem Durchmesser des Halsbereichs entspricht. Zur Verbindung mit dem Halsbereich wird das Befestigungselement mit seinem offenen Wandbereich radial an den Halsbereich angelegt und radial belastet, wobei das Befestigungselement elastisch aufgeweitet und letztlich mit dem Halsbereich des Infusionsbehälters verrastet wird.

In weiterer Ausgestaltung der Erfindung ist das zweite Befestigungselement derart gestaltet, dass es mit einer Tropfkammer des Infusionsgeräts verbindbar ist. Vorteilhafterweise ist das zweite Befestigungselement mit einem Wandabschnitt der im Wesentlichen zylinderförmigen Tropfkammerwandung verbindbar, welcher radial von der Tropfkammerwandung abragt. Derart kann eine besonders leicht herstellbare Verbindung zwischen der Sicherungsvorrichtung und dem Infusionsgerät erreicht werden.

In weiterer Ausgestaltung der Erfindung weist das Verbindungselement zumindest eine zwischen dem ersten Befestigungselement und dem zweiten Befestigungselement erstreckte Strebe auf. Vorteilhafterweise erstreckt sich die Strebe in einem befestigten Zustand der Fixiervorrichtung im Wesentlichen längs der Fixierrichtung. Um eine besonders zuverlässige Verbindung der Befestigungselemente aneinander zu gewährleisten, weist das Verbindungselement vorteilhafterweise zwei Streben auf.

In weiterer Ausgestaltung der Erfindung ist das Verbindungselement zumindest abschnittsweise elastisch dehnbar. Derart kann eine nicht fluchtende Ausrichtung des Einstechdorns in dem Einstechbereich oder ein Übermaß längs der Sicherungsrichtung durch ein nicht vollständiges Eintauchen des Einstechdorns bei der Verbindung der Sicherungsvorrichtung durch eine entsprechende Dehnung des Verbindungselements ausgeglichen werden. Dies bewirkt eine besonders zuverlässige und leicht herstellbare Sicherung.

In weiterer Ausgestaltung der Erfindung sind das erste Befestigungselement und das zweite Befestigungselement sowie das Verbindungselement einstückig zusammenhängend ausgebildet. Vorteilhafterweise ist die Sicherungsvorrichtung als Spritzgussteil, insbesondere aus Polypropylen, ausgebildet. Derart können eine kostengünstige Fertigung und vorteilhafte mechanische Eigenschaften erreicht werden.

Die Erfindung betrifft auch eine Infusionsanordnung mit einer Tropfkammer und/oder einem Infusionsbehälter sowie einer Sicherungsvorrichtung, die gemäß den vorstehenden Ausführungen gestaltet ist.

Weitere Vorteile und Merkmale der Erfindung ergeben sich aus den Ansprüchen und aus der nachfolgenden Beschreibung eines bevorzugten Ausführungsbeispiels der Erfindung, das anhand der Zeichnungen dargestellt ist.
- Fig. 1: zeigt in schematischer Seitenansicht einen Ausschnitt einer Infusionsanordnung im Bereich der Tropfkammer, welche mit einer bevorzugten Ausführungsform einer erfindungsgemäßen Sicherungsvorrichtung gesichert ist,
- Fig. 2: in einer Seitenansicht die Sicherungsvorrichtung nach Fig. 1,
- Fig. 3: eine weitere Ansicht der Sicherungsvorrichtung nach Fig. 1 und Fig. 2 längs einer Schnittlinie A-A gemäß Fig. 2 und
- Fig. 4: eine teilweise geschnittene Ansicht der Infusionsanordnung nebst Sicherungsvorrichtung nach Fig. 1 längs einer Schnittlinie B-B gemäß Fig. 3.

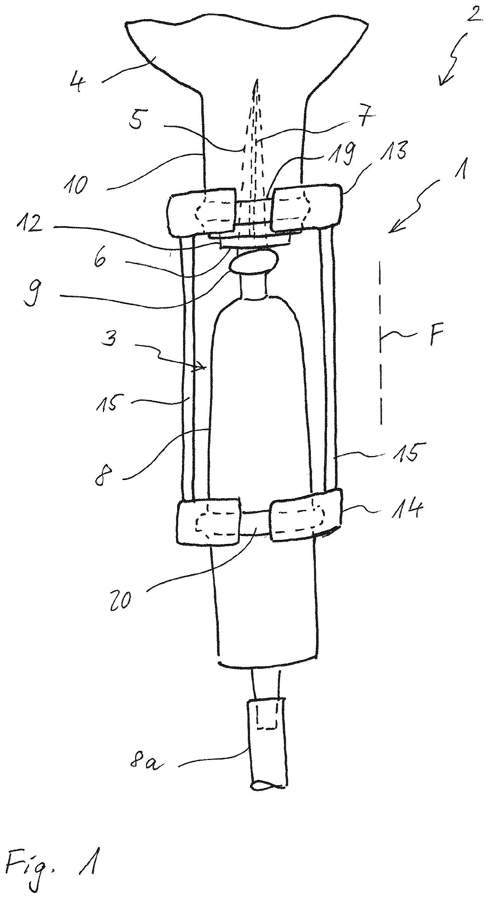

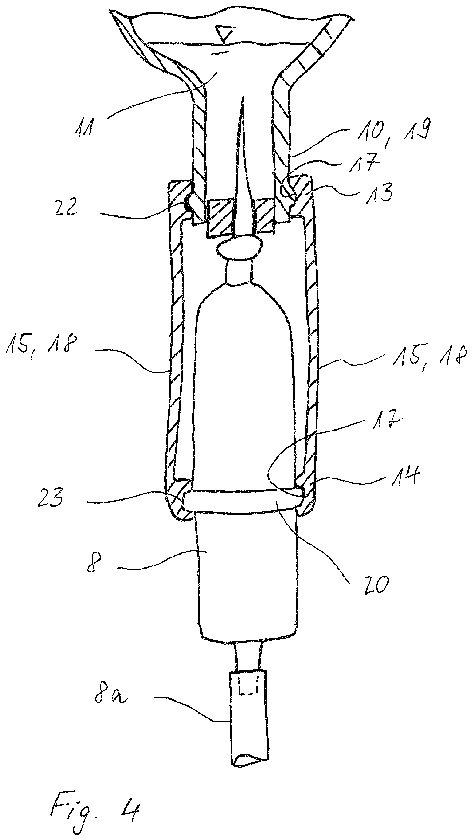

Eine Sicherungsvorrichtung 1 nach Fig. 1 bis 4 ist zur Sicherung einer Infusionsanordnung 2, wie sie anhand der Fig. 1 und 4 ersichtlich ist, vorgesehen. Die im Bereich der Medizintechnik als solche bekannte Infusionsanordnung 2 ist aus einem Infusionsgerät 3 und einem Infusionsbehälter 4 gebildet, wobei in den Fig. 1 und 4 sowohl das Infusionsgerät 3 als auch der Infusionsbehälter 4 aus Gründen der Übersichtlichkeit lediglich abschnittsweise dargestellt sind.

Das Infusionsgerät 3, das auch als Infusionssystem bezeichnet wird, dient zur flüssigkeitsleitenden Verbindung des Infusionsbehälters 4 mit einem nicht näher dargestellten patientenseitigen Zugang. Beispielsweise im Rahmen einer Druck- oder Schwerkraftinfusionstherapie kann durch die derartige Leitungsverbindung Infusionsflüssigkeit aus dem Infusionsbehälter 4 in den patientenseitigen Zugang eingeleitet werden. Zu diesem Zweck weist das Infusionsgerät 3 einen stirnseitig angeordneten Einstechdorn 5 auf, der zum Einstechen in einen Einstechbereich 6 des Infusionsbehälters 4 vorbereitet ist. Der Einstechdorn 5 weist eine längserstreckte Durchgangsbohrung 7 auf, mittels derer Infusionsflüssigkeit aus dem Inneren des Infusionsbehälters 4 ausgeleitet werden kann. Zudem weist das Infusionsgerät 3 eine Tropfkammer 8 auf, die in Längsrichtung des Infusionsgeräts 3 stirnseitig an den Einstechdorn 5 angrenzt und zur tropfenweisen Aufnahme von Infusionsflüssigkeit vorbereitet ist. Darüber hinaus weist das Infusionsgerät 3 eine zwischen dem Einstechdorn 5 und der Tropfkammer 8 angeordnete Belüftungseinrichtung 9 auf, die in den Fig. 1 und 4 lediglich schematisch angedeutet ist. Die Belüftungseinrichtung 9 ist zur kontrollierten Belüftung des Infusionsbehälters 4 bei der Ausleitung von Infusionsflüssigkeit vorgesehen. Ferner weist das Infusionsgerät 3 eine ausgangsseitig an der Tropfkammer 8 angeordnete Fluidleitung 8a in Gestalt eines Infusionsschlauchs auf, der aus Gründen der Übersichtlichkeit lediglich teilweise dargestellt ist. Ebenfalls nicht näher dargestellt, kann das Infusionsgerät 3 eine mit der Fluidleitung 8a in einer Wirkverbindung stehende Stelleinrichtung zur Einstellung des Flüssigkeitsdurchflusses durch die Fluidleitung 8a sowie einen ausgangseitig an der Fluidleitung 8a angeordneten Anschlusskonnektor zur Verbindung der Fluidleitung 8a mit dem patientenseitigen Zugang aufweisen.

Der an sich bekannte Infusionsbehälter 4 der Infusionsanordnung 2 ist in Form einer Flasche ausgebildet, wobei anhand der Fig. 1 und 4 lediglich ein Halsbereich 10 der Infusionsflasche ersichtlich ist. Ist der Infusionsbehälter 4 mit einer Infusionsflüssigkeit 11 befüllt, wie anhand Fig. 4 ersichtlich ist. Der Einstechbereich 6 des Infusionsbehälters 4 ist an einem elastischen Stopfen 12 vorgesehen. Der elastische Stopfen 12 dient zudem als Verschluss des Infusionsbehälters 4 und dichtet diesen in einem nichtdurchstochenen Zustand des Einstechbereichs 6 ab. Der elastische Stopfen 12 ist derart ausgestaltet, dass er von dem Einstechdorn 5 des Infusionsgeräts 3 von außen bis in das Innere des Infusionsbehälters 4 hineinreichend durchstochen werden kann.

Zum Herstellen der fluidleitenden Verbindung zwischen dem Infusionsbehälter 4 und dem, wie bereits beschrieben nicht näher dargestellten patientenseitigen Zugang, wird der Einstechdorn 5 des Infusionsgeräts 3 in den Einstechbereich 6 des elastischen Stopfens 12 des Infusionsbehälters 4 eingestochen. Sobald der elastische Stopfen 12 mittels des Einstechdorns 5 vollständig durchstoßen ist, ist eine fluidleitende Verbindung hergestellt, so dass Infusionsflüssigkeit 11 aus dem Inneren des Infusionsbehälters 4 durch die Durchgangsbohrung 7 in die Tropfkammer 8 und weiter in die Fluidleitung 8a bis zu dem patientenseitigen Zugang abgeleitet werden kann. Hierbei werden das Infusionsgerät 3 und der Infusionsbehälter 4 durch den zwischen dem Einstechdorn 5 und dem elastischen Stopfen 12 wirkenden Reibschluss mechanisch miteinander verbunden, d. h. gegeneinander fixiert, und bilden derart die Infusionsanordnung 2. Insbesondere um einem ungewollten Herausgleiten des Einstechdorns 5 aus dem elastischen Stopfen 12 entgegenzuwirken, ist die Sicherungsvorrichtung 1 vorgesehen.

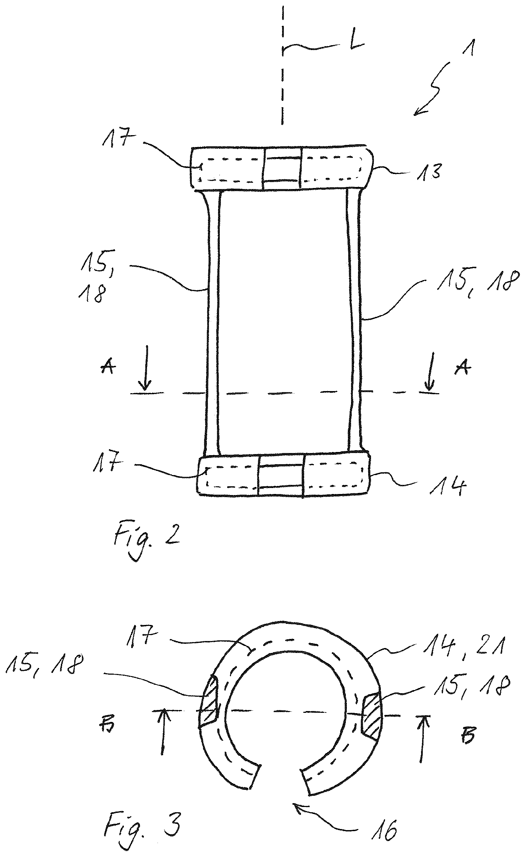

Wie unter anderem anhand der Fig. 2 ersichtlich ist, weist die Sicherungsvorrichtung 1 ein erstes Befestigungselement 13, ein zweites Befestigungselement 14 sowie zwei längserstreckte Verbindungselemente 15 auf, welche das erste Befestigungselement 13 und das zweite Befestigungselement 14 miteinander verbinden. Das erste Befestigungselement 13 und das zweite Befestigungselement 14 sind in Längsrichtung L der Sicherungsvorrichtung 1 übereinander und im Wesentlichen koaxial angeordnet. Wie anhand Fig. 3 ersichtlich ist, sind das erste Befestigungselement 13 und das zweite Befestigungselement 14 jeweils ringförmig ausgebildet und weisen jeweils einen in Umfangsrichtung offenen Wandbereich 16 auf, so dass sich im Wesentlichen eine C-förmige Gestalt der Befestigungselemente 13, 14. Infolgedessen ergibt sich eine leichte elastische Aufdehnbarkeit der Befestigungselemente 13, 14 in tangentialer Richtung. An ihren in radialer Richtung innenliegenden Flächen weisen die Befestigungselemente 13, 14 jeweils eine Profilierung 17 auf. Diese Profilierungen 17 sind jeweils in Gestalt einer Radialnut ausgebildet. Derart bilden die Befestigungselemente 13, 14 jeweils ein Rastelement 21, dass zum Herstellen einer kraft- und/oder formschlüssigen Verbindung vorgesehen ist.

Die Verbindungselemente 15 der Sicherungsvorrichtung 1 sind jeweils als Streben 18 ausgebildet. Diese Streben 18 erstrecken sich jeweils im Wesentlichen parallel zur Längsrichtung L von der Oberseite des zweiten Befestigungselementes 14 zu der Unterseite des ersten Befestigungselementes 13. Zudem weisen die Streben 18 jeweils eine im Vergleich zu den Befestigungselementen 13, 14 geringe Wandstärke auf, so dass die Streben 18 vergleichsweise leichter deformierbar sind. Infolge der bandförmigen Querschnittsgestaltung der Streben 18 wie sie anhand Fig. 3 ersichtlich ist, ergibt sich insbesondere eine elastische Dehnbarkeit der Streben 18 in Längsrichtung L. Zudem sind das erste Befestigungselement 13 und das zweite Befestigungselement 14 sowie die Streben 18 einstückig zusammenhängend in Gestalt eines Spritzgussteils ausgebildet.

Zum Sichern der Infusionsanordnung 2 mittels der Sicherungsvorrichtung 1 wird das erste Befestigungselement 13 mit seinem offenen Wandbereich 16 am Halsbereich 10 des Infusionsbehälters 4 angelegt, wobei die Längsachse L der Fixiervorrichtung 1 im Wesentlichen parallel zur Längserstreckung der Tropfkammer 8 angeordnet wird. Weiter wird das erste Befestigungselement 13 im Wesentlichen radial zum Halsbereich 10 belastet und derart tangential aufgeweitet, dass das erste Befestigungselement 13 in radialer Richtung vollständig über den Halsbereich 10 geschoben wird und mit diesem verrastet. Insoweit bildet der Halsbereich 10 einen Befestigungsbereich 19 des Infusionsbehälters 4. Wie anhand Fig. 4 ersichtlich ist, wird derart zwischen der Profilierung 17 an der Innenfläche des ersten Befestigungselements 13 und einer komplementären an dem Halsbereich 10 ausgebildeten Profilierung 22 eine form- sowie kraftschlüssige Verbindung bewirkt. Dabei umgreift das erste Befestigungselement 13 einen Abschnitt des Befestigungsbereichs 19 im Wesentlichen quer zur Sicherungsrichtung F von außen. Weiter wir das zweite Befestigungselement 14 mit der Tropfkammer 8 des Infusionsgeräts 3 verbunden. Zu diesem Zweck wird das zweite Befestigungselement 14 mit seinem offenen Wandbereich 16 an einen Befestigungsbereich 20 der Tropfkammer 8 angelegt. Weiter wird das zweite Befestigungselement 14 im Wesentlichen radial zur Längsrichtung der Tropfkammer 8 belastet, derart in tangentialer Richtung elastisch aufgeweitet, so dass das zweite Befestigungselement 14 vollständig über den Befestigungsbereich 20 geschoben wird und mit diesem verrastet. Dabei nimmt die an der Innenfläche des zweiten Befestigungselements 14 ausgebildete Profilierung 17 in Gestalt einer Nut eine zu dieser im Wesentlichen komplementär ausgebildete Profilierung 23 in Gestalt einer Wandaufragung zumindest abschnittsweise auf. Derart wird eine form- sowie kraftschlüssige Verbindung zwischen dem zweiten Befestigungselement 14 und der Tropfkammer 8 bewirkt. Dabei umgreift das zweite Befestigungselement 14 einen Abschnitt des Befestigungsbereichs 20 im Wesentlichen quer zur Sicherungsrichtung F von außen. Infolge der Sicherung der Infusionsanordnung 2 mittels der Sicherungsvorrichtung 1 wird einem ungewollten Herausziehen des Einstechdorns 5 des Infusionsgeräts 3 aus dem elastischen Stopfen 12 des Infusionsbehälters 4 entlang der Sicherungsrichtung F, welche im Wesentlichen der Längsrichtung L der Sicherungsvorrichtung entspricht, entgegengewirkt.

## Patentansprüche

1. Sicherungsvorrichtung (1) zur Sicherung eines an einem Infusionsbehälter (4) fixierten Infusionsgeräts (3) aufweisend ein erstes Befestigungselement (13), welches derart gestaltet ist, dass es mit einem Befestigungsbereich (19) des Infusionsbehälters (4) verbindbar ist, und ein zweites Befestigungselement (14), welches zu dem ersten Befestigungselement (13) beabstandet angeordnet und derart gestaltet ist, dass es mit einem Befestigungsbereich (20) des Infusionsgeräts (3) verbindbar ist, sowie mindestens ein Verbindungselement (15), welches das erste Befestigungselement (13) und das zweite Befestigungselement (14) miteinander verbindet, **dadurch gekennzeichnet, dass** das zweite Befestigungselement (14) derart gestaltet ist, dass es mit einer Tropfkammer (8) des Infusionsgeräts (3) verbindbar ist, wobei das zweite Befestigungselement (14) eine Profilierung (17) in Form einer Radialnut aufweist, die an einer in radialer Richtung innenliegenden Innenfläche ausgebildet und zur wenigstens abschnittsweisen Aufnahme einer im Wesentlichen komplementär ausgebildeten Profilierung (23) der Tropfkammer (8) vorgesehen ist.

2. Sicherungsvorrichtung (1) nach Anspruch 1, **dadurch gekennzeichnet, dass** zumindest eines der Befestigungselemente (13, 14) derart gestaltet ist, dass es den jeweiligen Befestigungsbereich (19, 20) in einem befestigten Zustand zumindest abschnittsweise und im Wesentlichen quer zu der Sicherungsrichtung (F) von außen umgreift.

3. Sicherungsvorrichtung (1) nach Anspruch 1 oder 2, **dadurch gekennzeichnet, dass** zumindest eines der Befestigungselemente (13, 14) derart gestaltet ist, dass es formschlüssig mit dem jeweiligen Befestigungsbereich verbindbar ist.

4. Sicherungsvorrichtung (1) nach einem der vorhergehenden Ansprüche, **dadurch gekennzeichnet, dass** zumindest eines der Befestigungselemente (13, 14) ein Rastelement (21) aufweist, welches mit dem jeweiligen Befestigungsbereich (19, 20) kraft- und/oder formschlüssig verbindbar ist.

5. Sicherungsvorrichtung (1) nach Anspruch 4, **dadurch gekennzeichnet, dass** das Rastelement (21) ringförmig ausgebildet ist und zumindest einen längs seiner Umfangsrichtung offenen Wandbereich (16) aufweist.

6. Sicherungsvorrichtung (1) nach einem der vorhergehenden Ansprüche, **dadurch gekennzeichnet, dass** das erste Befestigungselement (13) derart gestaltet ist, dass es mit einem Halsbereich (10) eines in Form einer Flasche ausgebildeten Infusionsbehälters (4) verbindbar ist.

7. Sicherungsvorrichtung (1) nach einem der vorhergehenden Ansprüche, **dadurch gekennzeichnet, dass** das Verbindungselement (15) zumindest eine zwischen dem ersten Befestigungselement (13) und dem zweiten Befestigungselement (14) erstreckte Strebe (18) aufweist.

8. Sicherungsvorrichtung (1) nach einem der vorhergehenden Ansprüche, **dadurch gekennzeichnet, dass** das Verbindungselement (15, 18, 18) zumindest abschnittsweise elastisch dehnbar ist.

9. Sicherungsvorrichtung (1) nach einem der vorhergehenden Ansprüche, **dadurch gekennzeichnet, dass** das erste Befestigungselement (13) und das zweite Befestigungselement (14) sowie das Verbindungselement (15, 18, 18) einstückig zusammenhängend ausgebildet sind.

10. Infusionsanordnung (2) mit einer Tropfkammer (8) und/oder einem Infusionsbehälter (4) sowie einer Sicherungsvorrichtung (1) nach einem der vorhergehenden Ansprüche.

## Claims

1. Securing device (1) for securing an infusion appliance (3) fixed to an infusion container (4), having a first fastening element (13), which is designed in such a way that it is connectable to a fastening region (19) of the infusion container (4), and a second fastening element (14), which is arranged at a distance from the first fastening element (13) and which is designed in such a way that it is connectable to a fastening region (20) of the infusion appliance (3), and at least one connection element (15), which connects the first fastening element (13) and the second fastening element (14) to each other, **characterized in that** the second fastening element (14) is designed in such a way that it is connectable to a drip chamber (8) of the infusion appliance (3), wherein the second fastening element (14) has a profile (17) in the form of a radial groove, which is formed on an interior inner face in the radial direction and is provided to receive at least a portion of a substantially complementary profile (23) of the drip chamber (8).

2. Securing device (1) according to claim 1, **characterized in that** at least one of the fastening elements (13, 14) is designed in such a way that, in a fastened state, it engages around the outside of the respective fastening region (19, 20) at least in part and substantially transversely with respect to the securing direction (F).

3. Securing device (1) according to claim 1 or 2, **characterized in that** at least one of the fastening elements (13, 14) is designed in such a way that it is connectable to the respective fastening region with form-fit engagement.

4. Securing device (1) according to one of the preceding claims, **characterized in that** at least one of the fastening elements (13, 14) has a locking element (21) which is connectable to the respective fastening region (19, 20) with force-fit and/or form-fit engagement.

5. Securing device (1) according to claim 4, **characterized in that** the locking element (21) is ring-shaped and has at least one open wall region (16) along its circumferential direction.

6. Securing device (1) according to one of the preceding claims, **characterized in that** the first fastening element (13) is designed in such a way that it is connectable to a neck region (10) of an infusion container (4) configured in the shape of a bottle.

7. Securing device (1) according to one of the preceding claims, **characterized in that** the connection element (15) has at least one strut (18) extending between the first fastening element (13) and the second fastening element (14).

8. Securing device (1) according to one of the preceding claims, **characterized in that** the connection element (15, 18, 18) is elastically extensible at least in part.

9. Securing device (1) according to one of the preceding claims, **characterized in that** the first fastening element (13) and the second fastening element (14) and the connection element (15, 18, 18) are formed contiguously in one piece.

10. Infusion arrangement (2) with a drip chamber (8) and/or an infusion container (4) and with a securing device (1) according to one of the preceding claims.

## Revendications

1. Dispositif de sécurisation (1) pour sécuriser un appareil de perfusion (3) fixé à un récipient de perfusion (4), présentant un premier élément de fixation (13) qui est configuré de manière à pouvoir être connecté à une région de fixation (19) du récipient de perfusion (4) et un deuxième élément de fixation (14) qui est disposé à distance du premier élément de fixation (13) et qui est configuré pour pouvoir être connecté à une région de fixation (20) de l'appareil de perfusion (3), ainsi qu'au moins un élément de connexion (15) qui relie l'un à l'autre le premier élément de fixation (13) et le deuxième élément de fixation (14), **caractérisé en ce que** le deuxième élément de fixation (14) est configuré de manière à pouvoir être connecté à une chambre de goutte-à-goutte (8) de l'appareil de perfusion (3), le deuxième élément de fixation (14) présentant un profilage (17) en forme de rainure radiale qui est réalisé au niveau d'une surface intérieure située à l'intérieur dans une direction radiale et qui est prévu pour recevoir au moins en partie un profilage (23) de la chambre de goutte-à-goutte (8) réalisé de manière essentiellement complémentaire.

2. Dispositif de sécurisation (1) selon la revendication 1, **caractérisé en ce qu'**au moins l'un des éléments de fixation (13, 14) est configuré de manière à venir en prise au moins en partie depuis l'extérieur autour de la région de fixation respective (19, 20), dans un état fixé et essentiellement transversalement à la direction de sécurisation (F).

3. Dispositif de sécurisation (1) selon la revendication 1 ou 2, **caractérisé en ce qu'**au moins l'un des éléments de fixation (13, 14) est configuré de manière à pouvoir être connecté par engagement par correspondance de formes avec la région de fixation respective.

4. Dispositif de sécurisation (1) selon l'une quelconque des revendications précédentes, **caractérisé en ce qu'**au moins l'un des éléments de fixation (13, 14) présente un élément d'encliquetage (21) qui peut être connecté par engagement par force et/ou par correspondance de formes à la région de fixation respective (19, 20).

5. Dispositif de sécurisation (1) selon la revendication 4, **caractérisé en ce que** l'élément d'encliquetage (21) est réalisé sous forme annulaire et présente au moins une région de paroi (16) ouverte le long de sa direction périphérique.

6. Dispositif de sécurisation (1) selon l'une quelconque des revendications précédentes, **caractérisé en ce que** le premier élément de fixation (13) est configuré de manière à pouvoir être connecté à une région de goulot (10) d'un récipient de perfusion (4) réalisé en forme de bouteille.

7. Dispositif de sécurisation (1) selon l'une quelconque des revendications précédentes, **caractérisé en ce que** l'élément de connexion (15) présente au moins une entretoise (18) s'étendant entre le premier élément de fixation (13) et le deuxième élément de fixation (14).

8. Dispositif de sécurisation (1) selon l'une quelconque des revendications précédentes, **caractérisé en ce que** l'élément de connexion (15, 18, 18) peut être étiré élastiquement au moins en partie.

9. Dispositif de sécurisation (1) selon l'une quelconque des revendications précédentes, **caractérisé en ce que** le premier élément de fixation (13) et le deuxième élément de fixation (14) ainsi que l'élément de connexion (15, 18, 18) sont réalisés de manière cohésive et d'une seule pièce.

10. Agencement de perfusion (2) comprenant une chambre de goutte-à-goutte (8) et/ou un récipient de perfusion (4) ainsi qu'un dispositif de sécurisation (1) selon l'une quelconque des revendications précédentes.
